# EUROPEAN PATENT APPLICATION

(11) **EP 1 953 233 A1**
(43) Date of publication of application: **06.08.2008**
(21) Application number: 07101628.1
(22) Date of filing: 02.02.2007
(51) Int. Cl.: C12P 7/42, C12P 7/62, C12P 17/06

(54) **Fermentation process for preparing pravastatin**

(71) Applicant: LEK Pharmaceuticals d.d., 61107 Ljubljana (SI)
(72) Inventor: The designation of the inventor has not yet been filed

(57) **Abstract**

The present invention relates to the improved production of pravastatin by using unpurified or partially purified raw materials or precursors.

## Description

### FIELD OF THE INVENTION

The invention in general relates to production of fermentation products using unpurified or partially purified raw materials or precursors. Raw materials or precursors for those fermentations are also gained from fermentations. More specifically the present invention from a field of pharmaceutics relates to a novel process for preparing pravastatin by means of a microbiological process.

### BACKGOUND OF THE INVENTION

Pravastatin and the salts thereof, in particular sodium salt are HMG-CoA reductase inhibitors disclosed in GB Pat. No. 1,555,831. Pravastatin is generally produced by two step fermentation, where first mevastatin is produced and isolated and added as a substrate to a medium where various microorganisms capable of converting mevastatin to pravastatin, usually of the genera *Gilbertella, Streptomyces, Circinella, Monascus, Nocardia, Amycolata, Mucor* or *Penicillium* are grown, which subsequently convert mevastatin into pravastatin, which is subsequently isolated by known methods. The microorganisms of above genera and specifically *Streptomyces Carbophylus, Saccharopolyspora hirsute, Amycolata autotrophica, Streptomyces carbophylus, Streptomyces californicus, Amycolata hydrocarbon-noxydans, Amycolatopsis fastidiosa, Streptomyces roseochromogenes* are accessible in public culture collections.

The fermentations are biotransformation processes where organisms are grown in medium while producing an industrially useful substance from simple precursors, like carbon and nitrogen sources, or converting a substance in their feed into another industrially useful substance.

### DISCLOSURE OF THE INVENTION

Mevastatin is preferably a product of fermenting a fungus *Penicillium citrinum* as accessible in public culture collections, such as *ATCC 38065,* while to produce pravastatin, one preferably ferments a bacteria *Streptomyces carbophylus* as accessible in public culture collections such as *SANK 62585.* The conditions to optimally grow fungi and bacteria , or in general mevastatin producing organisms and mevastatin-to-pravastatin converting organisms are usually different, and they require different media. The usual approach to produce pravastatin is thus to isolate mevastatin from first fermentation broth of first microorganism, usually in form of a salt and feed the solution of this salt to the second fermentation broth of second microorganism.

We have developed a process where by careful selection of media and fermentation condition we were able to dispose of the step of isolation of mevastatin from the first fermentation broth. That is very beneficial from economical point of view. In such procedure mevastatin can be added also in form of permeate, retentate, extract or similar.

An aspect of the invention is a process for preparing pravastatin which process comprises contacting fermentation broth (first fermentation broth) of a mevastatin producing microorganism (first organism) with a microorganism (second organism) capable of converting mevastatin to pravastatin.

In specific aspect said mevastatin producing microorganism is *Penicillium citrinum,* and a microorganism capable of converting mevastatin to pravastatin belongs the genus *Streptomyces,* more specifically it is *Streptomyces Carbophylus.*

In more specific aspect said first fermentation broth is sterilized prior to contacting with said second organism, and specifically this is done in second fermentation broth where the concentration of mevastatin is maintained below 3 g/L, preferably between 0,1 and 1 g/L.

In a process aspect the invention provides for preparation of pravastatin comprising steps:
culturing *Penicillium citrinum* in a first medium sustaining growth thereof under condition capable of producing mevastatin giving a fermentation broth containing mevastatin ;feeding said fermentation broth containing mevastatin to a second medium where a microorganism capable of converting mevastatin to pravastatin is grown; further growing said microorganism capable of converting mevastatin to pravastatin in said second medium under condition capable of producing pravastatin; and isolating pravastatin.

Specifically this is performed in a process which comprises steps:
culturing *Penicillium citrinum* in a first medium containing glycerol, glucose, yeast extract, soy meal, NaNO₃ , MgSO₄ at temperature around 25°C for up to 3 days giving a fermentation broth containing mevastatin; alkalizing said fermentation broth to pH between 10 and 10,5; (optionally) sterilizing said fermentation broth; (optionally) cooling said sterilized fermentation broth to below 30°C; (optionally) acidifying said fermentation broth to pH around 8 (where this step can be before or after sterilizing and/or cooling step); feeding said sterilized fermentation broth containing mevastatin to a second medium containing glucose, glycerol, soy meal, cornsteep liquor and cornsteep powder, cotton seed meal, and yeast extract where a microorganism *Streptomyces carbophylus* has been grown;
further growing said microorganism capable of converting mevastatin to pravastatin in said second medium at temperature around 28°C while controlling the pH of the fermentation broth to be in the range between 7.2 and 8 by addition of an aqueous solution of glucose; and isolating pravastatin.

Alternative aspect of the invention relates to the growth of an organism capable of converting mevastatin to pravastatin, preferably *Streptomyces carbophylus* in medium comprising glucose characterized by that after glucose has been consumed, to said medium the fermentation broth containing mevastatin is added continuously or in batches in a manner that the concentration of mevastatin in said broth is between 0.1 and 3g/L.

Aspect of the invention is also the use of the broth, specifically use of sterilized broth, or alkalized broth or alkalized, sterilized, cooled and acidified broth, produced during fermentation of *Penicillium citrinum* under condition capable of producing mevastatin, as a substrate in fermentation of a microorganism capable of converting mevastatin to pravastatin, specifically where a microorganism capable of converting mevastatin to pravastatin is selected from *Saccharopolyspora hirsute, Amycolata autotrophica, Streptomyces carbophylus (preferred), Streptomyces californicus, Amycolata hydrocarbon-noxydans, Amycolatopsis fastidiosa, Streptomyces roseochromogenes.*

An aspect is a fermentation medium capable to sustain growth of bacteria (preferably *Streptomyces),* containing between 1 and 30%( preferably up to 10%) vol/vol of broth, (preferably containing at least 1% of mevastatin) produced during fermentation of *Penicillium citrinum* under condition capable of producing mevastatin.

In accordance with our invention mevastatin producing fungi is fermented in first medium containing carbon source such as glycerol, glucose, nitrogen source such as yeast extract, soy meal, minerals, antifoaming agent. Mevastatin containing broth of this fermentation is alkalized, optionally sterilized and cooled and added as feed to the second medium containing carbon source such as glucose; nitrogen source such as soy meal, proflo, cotton seed meal; antifoaming agent wherein bacteria able to hydroxylate mevastatin to pravastatin has been grown for some time. The concentration of mevastatin in said second medium is kept between 0.1 and 3g/L The pravastatin produced is isolated from this broth.

In specific embodiment a seed medium containing glycerol 0.5-5%, glucose 2-8%, yeast extract 1-3%, soy meal 2-8%, NaNO₃ 0.1-1%, MgSO₄ 0.1-0.5%, antifoaming agent 0.05-0.2% is inoculated with a culture of *Penicilium citrinum* for up to 120 hours at 24+/-2°C, whereupon the matured seed culture is grown in a prefermentor containing glycerol 0.5-5%, glucose 2-8%, yeast extract 1-3%, soy meal 2-8%, NaNO₃ 0.1-1%, MgSO₄ 0.1-0.5%, antifoaming agent 0.05-0.2%, where it is grown at 24+/-2°C for up to 50 hours and from preferementor added to a fermentor containing sucrose 5-30%, glycerol 0-10%, glucose 0-30%, yeast extract 0-1.5%, soy meal 1-6%, cornsteep liquor/cornsteep powder 0-15%, NaNO₃ 0-0.3%, ZnSO₄ 0-0.1%, antifoaming agent 0.05-0.2% where it is grown at 24+/-2 °C for up to 350 hours.

To increase yield during the fermentation to the medium is continuously added an aqueous solution of carbon source such as sucrose, glucose, glycerol.

The amount of produced mevastatin is monitored by HPLC and the fermentation is stopped when the concentration is 10 to 25g/L,

The mevastatin containing broth is thereafter added an alkali (NaOH) to pH 10 -11,0, optionally sterilized at 121 °C for half hour and cooled. Thereafter or before the sterilisation the pH of said broth is optionally adjusted to around 8.

At the same time medium in prefermentor containing glucose 0-3%, glycerol 0-3%, soy mea10.5-1.5, yeast extract 0.1-1% and antifoaming agent 0.05-0.2% is inoculated with a culture of *Streptomyces carbophilus* and grown for up to 80 hours at 28+/-2°C. In case of large scale production also two step seed phase can be used. Where in first propagator phase the same seed medium as for prefermentor is used. After about 50 h cultivation at 28+/-2°C the culture is transferred to prefermentor phase. The prefermentor phase in case of two step process the prefermentor phase is shorter (up to 40h). In both cases the grown biomass is transferred to fermentor containing glucose 0-2%, glycerol 0-2%, soy meal 1-4%, CSL (cornsteep liquor)/CSP (cornsteep powder) 0-0.5%, cotton seed meal 0-0.5%, yeast extract 0-0.5%, antifoaming agent 0.05-0.2% and grown for about 15 to 40 hours at 28±2 °C. During the growth the pH is monitored and upon raise of pH the cooled above mevastatin containing broth is continuously or in batches added to the fermentor, taking care that the concentration of mevastatin in fermentor is bellow 3 g/I, more preferably 1 g/l. The fermentation is continued for up to 160 hours, where an aqueous solution of glucose is added to maintain the pH of fermentation broth between 7,2 and 8,2. Alternatively if the pH of mevastatin contained broth is not adjusted to around 8, an acid is used to control the pH.

During the fermentation mevastatin is converted to pravastatin, with final yield up to 6g/L, where the final amount of mevastatin is below 0.1 g/L, and after the fermentation the pravastatin is isolated from the broth.

In the first step of isolation the fermentation broth is charged to the microfiltration unit, where the mycelium is separated from the filtrate. The permeate is led to reverse osmosis unit to be additionally concentrated. The obtained concentrate (retentate) is led to extraction, where it is acidified and extracted to organic solvent. The obtained Pravastatin extract is collected and concentrated.
After concentration the tert butyl amine is added to obtain Pravastatin TBA salt. Some solvents, like water, methanol, acetone,... may be added to obtain pure Pravastatin TBA crystals. The obtained crystals are filtered and dried, and if desired converted to free acid or sodium salt.

### Example

A seed medium containing glycerol, glucose, yeast extract, soy meal, NaNO₃, MgSO₄ is inoculated with a culture of *Penicilium citrinum* for 2 days hours at 25°C, whereupon the matured seed culture is grown in a refermentor containing same medium, where it is grown same temperature for another 2 days and from preferementor added to a fermentor containing sucrose, glycerol, glucose, yeast extract, soy meal, cornsteep liquor/cornsteep powder, NaNO₃, ZnSO₄, where it is grown at 25 °C for 10 days. During the fermentation to the medium is continuously added an aqueous solution of sacharose.

The mevastatin containing broth is thereafter added an alkali to pH 10 -10,5, sterilized at 121 °C for half hour and cooled.

At the same time medium in prefermentor containing glucose, glycerol, soy meal, and yeast extract is inoculated with a culture of Streptomyces Carbophylus and grown for 3 days at 30°C. Thereafter the grown biomass is transferred to fermentor containing glucose, glycerol, soy meal, CSL /CSP, cotton seed meal, and yeast extract and grown for one day at 28±2 °C. Upon raise of pH from around 6,5 to above 7,1, which indicates that glucose has been consumed the above cooled mevastatin containing broth is in batches added to the fermentor, taking care that the concentration of mevastatin in fermentor is bellow 1 g/l. The fermentation is continued for 5 days maintaining the pH of fermentation broth between 7,2 and 8 with aqueous solution of glucose .

After the fermentation the pravastatin is isolated from the broth, which is first charged to the microfiltration unit, where the mycelium is separated from the filtrate which is acidified and extracted to ethyl acetate. After concentration the tert butyl amine is added to obtain Pravastatin tert butyl ammonium salt, which is isolated, and trans-salified to sodium salt.

## Claims

1. A process for preparing pravastatin which process comprises contacting fermentation broth of a mevastatin producing microorganism with a microorganism capable of converting mevastatin to pravastatin.

2. A process according to previous claim, where mevastatin producing microorganism is *Penicillium citrinum,* and a microorganism capable of converting mevastatin to pravastatin belongs the genus *Streptomyces.*

3. A process according to any of previous two claims, where mevastatin producing microorganism fermentation broth contains mevastatin in concentration from 10 to 25 g/L and said fermentation broth has been sterilized prior to contacting with a microorganism capable of converting mevastatin to pravastatin.

4. A process according to any of previous 3 claims, where in the medium in which fermentation broth of a mevastatin producing microorganism is contacted with microorganism of the genus *Streptomyces,* the concentration of mevastatin is maintained below 3 g/L

5. A process according to any of previous 4 claims, where a microorganism of the genus *Streptomyces* is *Streptomyces carbophylus.*

6. A process for preparation of pravastatin which comprises steps:
a.) culturing *Penicillium citrinum* in a first medium sustaining growth thereof under condition capable of producing mevastatin giving a fermentation broth containing mevastatin;
b.) feeding said fermentation broth containing mevastatin to a second medium where a microorganism capable of converting mevastatin to pravastatin is grown;
c.) further growing said microorganism capable of converting mevastatin to pravastatin in said second medium under condition capable of producing pravastatin; and
d.) isolating pravastatin.

7. A process for preparation of pravastatin according to previous claim which comprises steps:
a.) culturing *Penicillium citrinum* in a first medium containing glycerol, glucose, yeast extract, soy meal, NaNO₃ , MgSO₄ at temperature around 25°C for up to 3 days giving a fermentation broth containing mevastatin;
b.) alkalizing said fermentation broth to pH between 10 and 10,5;
c.) (optionally) sterilizing said fermentation broth;
d.) (optionally) cooling said sterilized fermentation broth to below 30°C
e.) (optionally) acidifying said fermentation broth to pH around 8;
f.) feeding said sterilized fermentation broth containing mevastatin to a second medium containing glucose, glycerol, soy meal, cornsteep liquor and cornsteep powder, cotton seed meal, and yeast extract where a microorganism *Streptomyces carbophylus* has been grown;
g.) further growing said microorganism capable of converting mevastatin to pravastatin in said second medium at temperature around 28°C while controlling the pH of the fermentation broth to be in the range between 7.2 and 8 by addition of an aqueous solution of glucose or acid ; and
h.) isolating pravastatin.

8. A process for growing an organism capable of converting mevastatin to pravastatin in medium comprising glucose **characterized by** that after glucose has been consumed, to said medium the fermentation broth containing mevastatin is added continuously or in batches in a manner that the concentration of mevastatin in said broth is between 0.1 and 3g/L.

9. Use of the broth, produced during fermentation of *Penicillium citrinum* under condition capable of producing mevastatin, as a substrate in fermentation of a microorganism capable of converting mevastatin to pravastatin.

10. Use according to previous claim where a microorganism capable of converting mevastatin to pravastatin is selected from group consisting of, *Saccharopolyspora hirsute, Amycolata autotrophica, Streptomyces carbophylus, Streptomyces californicus, Amycolata hydrocarbon-noxydans, Amycolatopsis fastidiosa, Streptomyces roseochromogenes.*
